# EUROPEAN PATENT SPECIFICATION

(11) **EP 0 288 852 B1**
(45) Date of publication and mention of the grant of the patent: **28.07.1993**
(21) Application number: 88106108.9
(22) Date of filing: 16.04.1988
(51) Int. Cl.: C07D 219/10, C07D 221/16, C07D 401/12, C07D 405/12, C07D 409/12, A61K 31/435

(54) **9-Hydroxyamino tetrahydro-acridines, a process for their preparation and their use as medicaments**
9-Hydroxyamino-tetrahydroacridine, Verfahren zu ihrer Herstellung und ihre Verwendung als Medikamente
Hydroxyamino-9 tétrahydroacridines, procédé pour leur préparation et leur utilisation comme médicaments

(30) Priority: 20.04.1987 US 39883
(43) Date of publication of application: 02.11.1988
(73) Proprietor: HOECHST-ROUSSEL PHARMACEUTICALS INCORPORATED, Somerville New Jersey 08876 (US)
(72) Inventor: Shutske, Gregory Michael, Somerset, N.J. 08873 (US)
(74) Representative: Losert, Wolfgang Dr.

(56) References cited:
- EP-A- 0 179 383
- US-A- 3 318 896
- US-A- 4 550 113
- REMINGTON'S PHARMACEUTICAL SCIENCES, 15th ed., 1975, ch. 29, p. 466

## Description

This invention relates to compounds having the formula 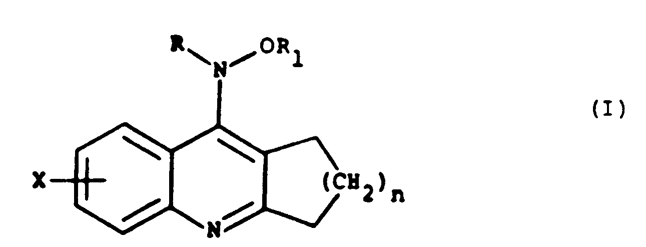
wherein n is 1-4; X is hydrogen, loweralkyl, cycloalkyl, loweralkoxy, halogen, hydroxy, nitro, trifluoromethyl formyl, loweralkylcarbonyl, arylcarbonyl, -SH, loweralkylthio, NHCOR₂ or -NR₃R₄ where R₂ is hydrogen or loweralkyl, and R₃ and R₄ are independently hydrogen, loweralkyl or cycloalkyl; R is hydrogen, loweralkyl or loweralkylcarbonyl; and R₁ is hydrogen, loweralkyl, aryl, diloweralkylaminoloweralkyl, arylloweralkyl, furylloweralkyl, thienylloweralkyl, pyridinylloweralkyl, diarylloweralkyl, oxygen-bridged arylloweralkyl or oxygen-bridged diarylloweralkyl; stereo, optical and geometrical isomers thereof, and pharmaceutically acceptable acid addition salts thereof which are useful for enhancing memory, a method for synthesizing them, and pharmaceutical compositions comprising an effective memory enhancing amount of such a compound.

Throughout the specification and the appended claims, a given chemical formula or name shall encompass all stereo and optical isomers thereof where such isomers exist, as well as pharmaceutically acceptable acid addition salts thereof and solvates thereof such as for instance hydrates.

The following definitions shall apply throughout the specification and the appended claims.

Unless otherwise stated or indicated, the term loweralkyl denotes a straight or branched alkyl group having from 1 to 6 carbon atoms. Examples of said alkyl include methyl, ethyl, n-propyl, iso-butyl, pentyl, and hexyl.

Unless otherwise stated or indicated, the term cycloalkyl denotes a saturated ring containing 3 to 7 carbon atoms. Examples of said cycloalkyl include cyclopropyl, cyclohexyl and cycloheptyl.

Unless otherwise stated or indicated, the term loweralkoxy denotes a straight or branched alkoxy group having 1 to 6 carbon atoms. Examples of said alkoxy include methoxy, ethoxy, iso-propoxy, sec-butoxy, and straight and branched chain hexyloxy.

Unless otherwise stated or indicated, the term halogen shall mean fluorine, chlorine, bromine or iodine.

Unless otherwise stated or indicated, the term aryl shall mean an unsubstituted phenyl group, a phenyl group substituted with 1, 2 or 3 substituents each of which being independently loweralkyl, loweralkoxy, halogen, hydroxy, trifluoromethyl, phenyl or benzyloxy.

Unless otherwise stated or indicated, the term oxygen-bridged shall signify the fact that an oxygen atom is present between aryl and loweralkyl groups and/or an oxygen atom has replaced a methylene group in the loweralkyl group, with the proviso that said methylene group is not alpha to the amino nitrogen carrying the groups R and R₁. Thus, for instance, examples of oxygen-bridged arylloweralkyl include 3-phenoxypropyl and 4-phenoxybutyl, and examples of oxygen-bridged diarylloweralkyl include
2-[bis(4-fluorophenyl)methoxy]ethyl and
2-[bis(3-fluorophenyl)methoxy]ethyl.

The compounds of this invention are prepared as described below. The required O-substituted hydroxylamines of formula II wherein R is hydrogen and R₁ is not hydrogen are, in some cases, commercially available and, where not, can readily be prepared by one skilled in the art by means of the alkylation and subsequent hydrolysis of N-hydroxyphthalimide as disclosed by A. Rougny and M. Daudon, Bull. Soc. Chim. France, 833 (1976). Further alkylation by methods known to one skilled in the art allows the preparation of the compounds of formula II wherein R is loweralkyl and R₁ is not hydrogen.

In order to simplify the description of the synthetic schemes, the description will be presented with specific reference to the situation where n=2, but it will readily be understood that the synthetic schemes can also be applied to the other situations by making obvious modifications where necessary.

EP-A-0 179 383 discloses 9-amino-1,2,3,4-tetrahydroacridin-1-ols and -1-ones and derivatives thereof, which are useful in the treatment of various memory dysfunctions characterized by decreased cholinergic function.

Throughout the description of the synthetic steps, definitions of X, R and R₁ through R₄ are as given above unless otherwise stated or indicated.

A compound of formula IV wherein R is not loweralkylcarbonyl can be prepared by reacting a compound of formula III with a hydroxylamine of formula II. Said reaction can be conducted at a temperature of 120̸-220̸°C in the presence of a hydroxylated aromatic compound such as phenol or cresol.

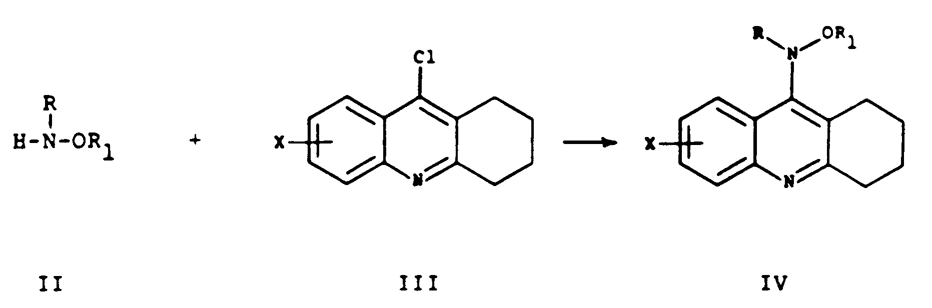

A compound of formula IVb wherein R is lower alkylcarbonyl can be prepared by reacting a compound of formula IVa with an acylating agent such as an acid halide or anhydride of formula V wherein R₅ is loweralkyl and Y is chlorine, bromine or OC(=O)R₅. The reaction can be carried out in an inert solvent such as chloroform, methylene chloride, toluene, tetrahydrofuran or diethyl ether in the presence of a proton acceptor such as pyridine, 4-dimethylaminopyridine, triethylamine or diisopropylethylamine at a temperature of 0̸-10̸0̸°C.

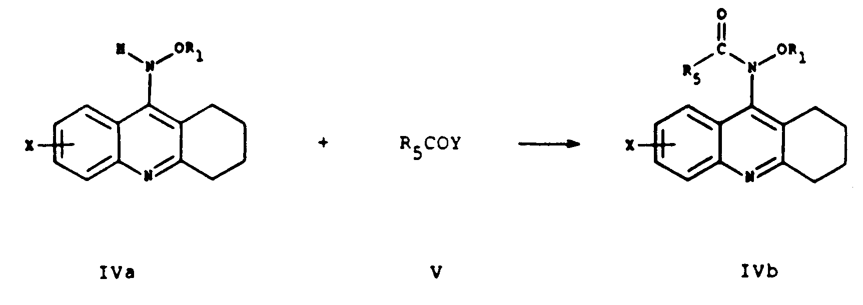

The compounds of Formula I of the present invention can be used for the treatment of various memory dysfunctions characterized by decreased cholinergic function, such as Alzheimer's disease.

This utility can be ascertained by determining the ability of these compounds to inhibit the activity of the enzyme acetylcholinesterase and thereby increase the acetylcholine levels in the brain.

This utility can also be ascertained by determining the ability of these compounds to restore cholinergically deficient memory in the Dark Avoidance Assay. In this assay mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

Effective quantities of the compounds of the invention may be administered to a patient by any of the various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0̸.5% of active compound, but may be varied depending upon the particular form and may conveniently be between 4% to about 70̸% of the weight of the unit. The amount of active compound in such compositions is such that a suitable dosage will be obtained. Preferred compositions and preparations according to the present invention are prepared so that an oral dosage unit form contains between 1.0̸-30̸0̸ milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administration, the active compounds of the invention may be incorporated into a solution or suspension. These preparations should contain at least 0̸.1% of active compound, but may be varied between 0̸.5 and about 30̸% of the weight thereof. The amount of active compound in such compositions is such that a suitable dosage will be obtained. Preferred compositions and preparations according to the present invention are prepared so that a parenteral dosage unit contains between 0̸.5 to 10̸0̸ milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in disposable syringes or multiple dose vials made of glass or plastic.

Examples of the compounds of this invention include:
N-hydroxy-1,2,3,4-tetrahydro-9-acridinamine;
6-chloro-N-hydroxy-1,2,3,4-tetrahydro-9-acridinamine;
N-hydroxy-7-methoxy-1,2,3,4-tetrahydro-9-acridinamine;
N-methoxy-1,2,3,4-tetrahydro-9-acridinamine;
N-ethoxy-7-methyl-1,2,3,4-tetrahydro-9-acridinamine;
N-(phenylmethoxy)-1,2,3,4-tetrahydro-9-acridinamine;
N-(2-furylmethoxy)-1,2,3,4-tetrahydro-9-acridinamine;
N-[(2-phenylethyl)oxy]-1,2,3,4-tetrahydro-9-acridinamine;
N-[(4,4-diphenylbutyl)oxy]-1,2,3,4-tetrahydro-9-acridinamine;
N-[(2-dimethylaminoethyl)oxy]-1,2,3,4-tetrahydro-9-acridinamine;
N-[(3-phenoxypropyl)oxy]-1,2,3,4-tetrahydro-9-acridinamine;
N-methoxy-7,8,9,10̸-tetrahydro-6H-cyclohepta[b]quinolin-11-amine; and
2,3-dihydro-N-(phenylmethoxy)-1H-cyclopenta[b]quinolin-9-amine.

## Claims (Claims for the following Contracting State(s): AT, BE, CH, DE, FR, GB, IT, LI, LU, NL, SE)

1. A compound of the formula I 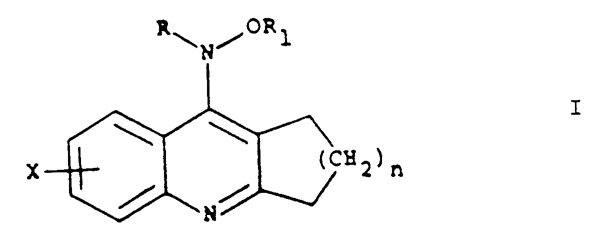 wherein n is 1-4; X is hydrogen, C₁-C₆-alkyl, C₃-C₇-cycloalkyl, C₁-C₆-alkoxy, halogen, hydroxy, nitro, trifluoromethyl formyl, C₁-C₆-alkylcarbonyl, phenylcarbonyl, wherein the phenyl group is unsubstituted or substituted with 1, 2 or 3 substituents each of which being independently C₁-C₆-alkyl, C₁-C₆-alkoxy, halogen, hydroxy, trifluoromethyl, phenyl or benzyloxy, -SH, C₁-C₆-alkylthio, NHCOR₂ or -NR₃R₄ where R₂ is hydrogen or C₁-C₆-alkyl, and R₃ and R₄ are indenpendently hydrogen, C₁-C₆-alkyl or C₃-C₇-cycloalkyl; R is hydrogen, C₁-C₆-alkyl or C₁-C₆-alkylcarbonyl; and R₁ is hydrogen, C₁-C₆-alkyl, di-C₁-C₆-alkylamino-C₁-C₆-alkyl, furyl-C₁-C₆-alkyl, thienyl-C₁-C₆-alkyl, pyridinyl-C₁-C₆-alkyl or phenyl, phenyl-C₁-C₆-alkyl, di-phenyl-C₁-C₆-alkyl, oxygen-bridged phenyl-C₁-C₆-alkyl or oxygen-bridged di-phenyl-C₁-C₆-alkyl wherein the phenyl group is unsubstituted or substituted with 1, 2 or 3 substituents each of which being independently C₁-C₆-alkyl C₁-C₆-alkoxy, halogen, hydroxy, trifluormethyl, phenyl or benzyloxy; a stereo, optical or geometrical isomer thereof, or a pharmaceutically acceptable acide addition salt thereof.

2. The compound as defined in Claim 1, where n is 2.

3. The compound as defined in Claim 2, where R is hydrogen and R₁ is hydrogen or phenyl-C₁-C₆-alkyl, wherein the phenyl group is unsubstituted or substituted with 1, 2 or 3 substituents each of which being independently C₁-C₆-alkyl C₁-C₆-alkoxy, halogen, hydroxy, trifluoromethyl, phenyl or benzyloxy.

4. The compound as defined in Claim 3, where X is hydrogen, C₁-C₆-alkyl or trifluoromethyl.

5. A process for the preparation of a compound as defined in claim 1 which comprises
a) reacting a compound of the formula III 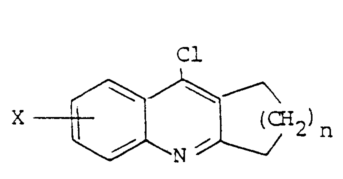 where X and n are as defined, with a hydroxylamine of formula II 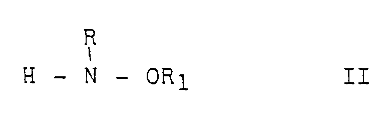 where R₁ is as defined and R is as defined but is not C₁-C₆-alkylcarbonyl, and
b) optionally reacting a compound of formula I where R is as defined but is not C₁-C₆-alkylcarbonyl with an acylating agent to afford a compound of formula I where R is C₁-C₆-alkylcarbonyl, and
optionally preparing in a customary manner a pharmaceutically acceptable acid addition salt thereof.

6. A pharmaceutical composition which comprises as the active ingredient a compound as defined in claim 1 and a pharmaceutically acceptable carrier.

7. Use of a compound as defined in claim 1 for the preparation of a medicament having cholinergic function enhancing activity.

## Claims (Claims for the following Contracting State(s): ES, GR)

1. A process for the preparation of a compound of the formula I 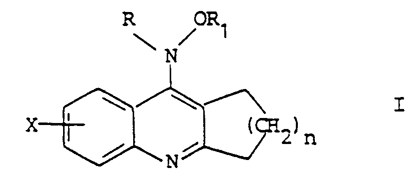 wherein n is 1-4; X is hydrogen, C₁-C₆-alkyl, C₃-C₇-cycloalkyl, C₁-C₆-alkoxy, halogen, hydroxy, nitro, trifluoromethyl formyl, C₁-C₆-alkylcarbonyl, phenylcarbonyl, wherein the phenyl group is unsubstituted or substituted with 1, 2 or 3 substituents each of which being independently C₁-C₆-alkyl, C₁-C₆-alkoxy, halogen, hydroxy, trifluoromethyl, phenyl or benzyloxy, -SH, C₁-C₆-alkylthio, NHCOR₂ or -NR₃R₄ where R₂ is hydrogen or C₁-C₆-alkyl, and R₃ and R₄ are indenpendently hydrogen, C₁-C₆-alkyl or C₃-C₇-cycloalkyl; R is hydrogen, C₁-C₆-alkyl or C₁-C₆-alkylcarbonyl; and R₁ is hydrogen C₁-C₆-alkyl, di-C₁-C₆-alkylamino-C₁-C₆-alkyl, furyl-C₁-C₆-alkyl, thienyl-C₁-C₆-alkyl, pyridinyl-C₁-C₆-alkyl or phenyl, phenyl-C₁-C₆-alkyl, di-phenyl-C₁-C₆-alkyl, oxygen-bridged phenyl-C₁-C₆-alkyl or oxygen-bridged di-phenyl-C₁-C₆-alkyl wherein the phenyl group is unsubstituted or substituted with 1, 2 or 3 substituentseach of which being independently C₁-C₆-alkyl C₁-C₆-alkoxy, halogen, hydroxy, trifluormethyl, phenyl or benzyloxy; a stereo, optical or geometrical isomer thereof, or a pharmaceutically acceptable acide addition salt thereof,
which comprises
a) reacting a compound of the formula III 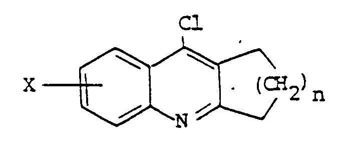 where X and n are as defined, with a hydroxylamine of formula II 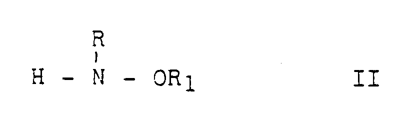 where R₁ is as defined and R is as defined but is not C₁-C₆-alkylcarbonyl, and
b) optionally reacting a compound of formula I where R is as defined but is not C₁-C₆-alkylcarbonyl with an acylating agent to afford a compound of formula I where R is C₁-C₆-alkylcarbonyl, and
optionally preparing in a customary manner a pharmaceutically acceptable acid addition salt thereof.

2. A process as defined in claim 1, where n is 2.

3. A process as defined in claim 2, where n is hydrogen and R₁ is hydrogen or phenyl-C₁-C₆-alkyl.

4. A process as defined in claim 3, where X is hydrogen, C₁-C₆-alkyl or trifluoromethyl.

5. Use of a compound as defined in claim 1 for the preparation of a medicament having cholinergic function enhancing activity.

## Patentansprüche (Patentansprüche für folgende(n) Vertragsstaat(en): AT, BE, CH, DE, FR, GB, IT, LI, LU, NL, SE)

1. Verbindung mit der Formel I 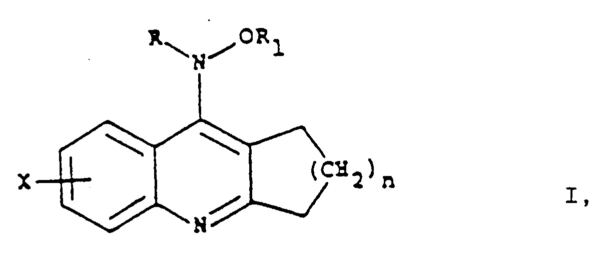 worin n 1 - 4 ist; X für Wasserstoff, C₁-C₆-Alkyl, C₃-C₇-Cycloalkyl,C₁-C₆-Alkoxy, Halogen, Hydroxy, Nitro, Trifluormethyl, Formyl, C₁-C₆-Alkylcarbonyl, Phenylcarbonyl, worin die Phenylgruppe unsubstituiert oder durch 1, 2 oder 3 Substituenten substituiert ist, von denen jeder unabhängig C₁-C₆-Alkyl, C₁-C₆-Alkoxy, Halogen, Hydroxy, Trifluormethyl, Phenyl oder Benzyloxy ist, -SH, C₁-C₆-Alkylthio, NHCOR₂ oder -NR₃R₄ steht, worin R₂ Wasserstoff oder C₁-C₆-Alkyl bedeutet und R₃ und R₄ unabhängig voneinander Wasserstoff, C₁-C₆-Alkyl oder C₃-C₇-Cycloalkyl bedeuten; R für Wasserstoff, C₁-C₆-Alkyl oder C₁-C₆-Alkylcarbonyl steht; und R₁ Wasserstoff, C₁-C₆-Alkyl, Di-C₁-C₆-alkylamino-C₁-C₆-alkyl, Furyl-C₁-C₆-alkyl, Thienyl-C₁-C₆-alkyl, Pyridinyl-C₁-C₆-alkyl oder Phenyl, Phenyl-C₁-C₆-alkyl, Diphenyl-C₁-C₆-alkyl, sauerstoffverbrücktes Phenyl-C₁-C₆-alkyl oder sauerstoffverbrücktes Diphenyl-C₁-C₆-alkyl bedeutet, worin die Phenylgruppe unsubstituiert oder durch 1, 2 oder 3 Substituenten substituiert ist, von denen jeder unabhängig C₁-C₆-Alkyl C₁-C₆-Alkoxy, Halogen, Hydroxy, Trifluormethyl, Phenyl oder Benzyloxy bedeutet; ein stereomeres, optisches oder geometrisches Isomer hievon oder ein pharmazeutisch annehmbares Säureadditionssalz hievon.

2. Verbindung nach Anspruch 1, worin n den Wert 2 hat.

3. Verbindung nach Anspruch 2, worin R Wasserstoff bedeutet und R₁ für Wasserstoff oder Phenyl-C₁-C₆-alkyl steht, worin die Phenylgruppe unsubstituiert oder durch 1, 2 oder 3 Substituenten substituiert ist, von denen jeder unabhängig C₁-C₆-Alkyl, C₁-C₆Alkoxy, Halogen, Hydroxy, Trifluormethyl, Phenyl oder Benzyloxy ist.

4. Verbindung nach Anspruch 3, worin X für Wasserstoff, C₁-C₆-Alkyl oder Trifluormethyl steht.

5. Verfahren zur Herstellung einer Verbindung nach Anspruch 1, welches umfaßt:
a) Umsetzen einer Verbindung der Formel III 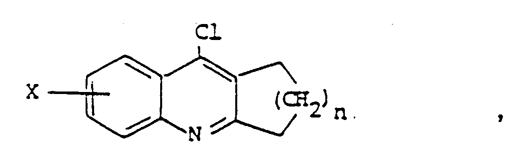 worin X und n wie definiert sind, mit einem Hydroxylamin der Formel II 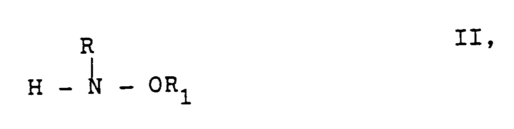 worin R₁ wie definiert ist und R die obige Definition aufweist, nicht aber für C₁-C₆-Alkylcarbonyl steht, und
b) gewünschtenfalls Umsetzen einer Verbindung der Formel I, worin R wie definiert ist, nicht aber für C₁-C₆-Alkylcarbonyl steht, mit einem Acylierungsmittel, um eine Verbindung der Formel I zu erhalten, worin R für C₁-C₆-Alkylcarbonyl steht, und
gewünschtenfalls Herstellen eines pharmazeutisch annehmbaren Säureadditionssalzes hievon in üblicher Weise.

6. Pharmaseutische Zusammensetzung, die als wirksamen Bestandteil eine Verbindung nach Anspruch 1 und einen pharmazeutisch annehmbaren Träger umfaßt.

7. Verwendung einer Verbindung nach Anspruch 1 zur Herstellung eines Medikaments mit einer die cholinerge Funktion fördernden Aktivität.

## Patentansprüche (Patentansprüche für folgende(n) Vertragsstaat(en): ES, GR)

1. Verfahren zur Herstellung einer Verbindung mit der Formel I 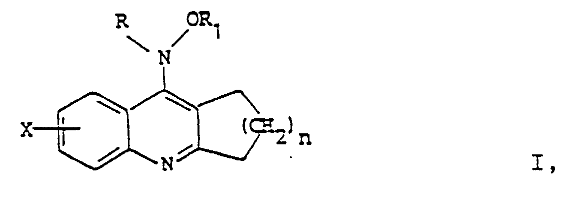 worin n 1 - 4 ist; X für Wasserstoff, C₁-C₆-Alkyl, C₃-C₇-Cycloalkyl, C₁-C₆-Alkoxy, Halogen, Hydroxy, Nitro, Trifluormethyl, Formyl, C₁-C₆-Alkylcarbonyl, Phenylcarbonyl, worin die Phenylgruppe unsubstituiert oder durch 1, 2 oder 3 Substituenten substituiert ist, von denen jeder unabhängig C₁-C₆-Alkyl, C₁-C₆-Alkoxy, Halogen, Hydroxy, Trifluormethyl, Phenyl oder Benzyloxy ist, -SH, C₁-C₆-Alkylthio, NHCOR₂ oder -NR₃R₄ steht, worin R₂ Wasserstoff oder C₁-C₆-Alkyl bedeutet und R₃ und R₄ unabhängig voneinander Wasserstoff, C₁-C₆-Alkyl oder C₃-C₇-Cycloalkyl bedeuten; R für Wasserstoff, C₁-C₆-Alkyl oder C₁-C₆-Alkylcarbonyl steht; und R₁ Wasserstoff, C₁-C₆-Alkyl, Di-C₁-C₆-alkylamino-C₁-C₆-alkyl, Furyl-C₁-C₆-alkyl, Thienyl-C₁-C₆-alkyl, Pyridinyl-C₁-C₆-alkyl oder Phenyl, Phenyl-C₁-C₆-alkyl, Diphenyl-C₁-C₆-alkyl, sauerstoffverbrücktes Phenyl-C₁-C₆-alkyl oder sauerstoffverbrücktes Diphenyl-C₁-C₆-alkyl bedeutet, worin die Phenylgruppe unsubstituiert oder durch 1, 2 oder 3 Substituenten substituiert ist, von denen jeder unabhängig C₁-C₆-Alkyl C₁-C₆-Alkoxy, Halogen, Hydroxy, Trifluormethyl, Phenyl oder Benzyloxy bedeutet; eines stereomeren, optischen oder geometrischen Isomeren hievon oder eines pharmazeutisch annehmbaren Säureadditionssalzes hievon, welches Verfahren umfaßt
a) Umsetzen einer Verbindung der Formel III 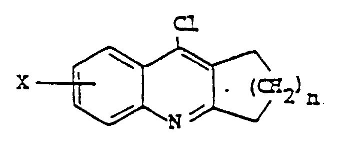 worin X und n wie definiert sind, mit einem Hydroxylamin der Formel II 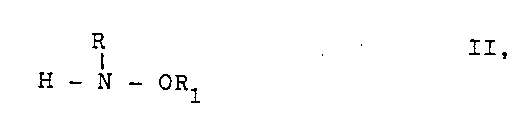 worin R₁ wie definiert ist und R die obige Definition aufweist, nicht aber für C₁-C₆-Alkylcarbonyl steht, und
b) gewünschtenfalls Umsetzen einer Verbindung der Formel I, worin R wie definiert ist, nicht aber für C₁-C₆-Alkylcarbonyl steht, mit einem Acylierungsmittel, um eine Verbindung der Formel I zu erhalten, worin R für C₁-C₆-Alkylcarbonyl steht, und
gewünschtenfalls Herstellen eines pharmazeutisch annehmbaren Säureadditionssalzes hievon in üblicher Weise.

2. Verfahren nach Anspruch 1, worin n den Wert 2 hat.

3. Verfahren nach Anspurch 2, worin n Wasserstoff bedeutet und R₁ für Wasserstoff oder Phenyl-C₁-C₆-alkyl steht.

4. Verfahren nach Anspruch 3, worin X für Wasserstoff, C₁-C₆-Alkyl oder Trifluormethyl steht.

5. Verwendung einer Verbindung nach Anspruch 1 zur Herstellung eines Medikamentes mit einer die cholinerge Funktion fördernden Aktivität.

## Revendications (Revendications pour l'(les) Etat(s) contractant(s) suivant(s): AT, BE, CH, DE, FR, GB, IT, LI, LU, NL, SE)

1. Composé de formule I 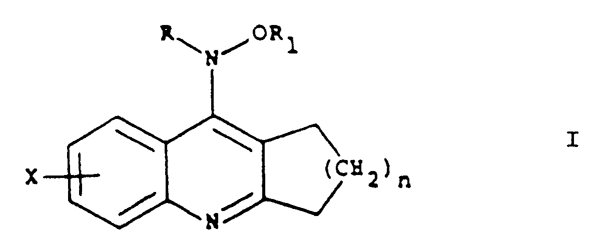 dans laquelle n est 1-4; X est un atome d'hydrogène ou d'halogène ou un radical alkyle en C₁-C₆, cycloalkyle en C₃-C₇, alcoxy en C₁-C₆, hydroxy, nitro, trifluorométhyle, formyle, alkyl(C₁-C₆)-carbonyle, phénylcarbonyle dans lequel le fragment phényle est non substitué ou substitué par 1, 2 ou 3 substituants, chacun étant indépendamment un atome d'halogène ou un groupe alkyle en C₁-C₆, alcoxy en C₁-C₆, hydroxy, trifluorométhyle, phényle ou benzyloxy, un radical -SH, alkyl(C₁-C₆)-thio_{,} NHCOR₂ ou -NR₃R₄, R₂ étant un atome d'hydrogène ou un groupe alkyle en C₁-C₆, et R₃ et R₄ étant indépendamment un atome d'hydrogène ou un groupe alkyle en C₁-C₆ ou cycloalkyle en C₃-C₇; R est un atome d'hydrogène ou un groupe alkyle en C₁-C₆ ou alkyl(C₁-C₆)-carbonyle; et R₁ est un atome d'hydrogène ou un radical alkyle en C₁-C₆, dialkyl(C₁-C₆)-amino-alkyle(C₁-C₆), furyl-alkyle(C₁-C₆), thiényl-alkyle(C₁-C₆), pyridinyl-alkyle(C₁-C₆) ou phényle, phényl-alkyle(C₁-C₆), diphényl-alkyle(C₁-C₆), un radical phényl-alkyle(C₁-C₆) ponté par un atome d'oxygène ou un radical diphényl-alkyle(C₁-C₆) ponté par un atome d'oxygène, le fragment phényle étant non substitué ou substitué par 1, 2 ou 3 substituants, chacun étant indépendamment un atome d'halogène ou un radical alkyle en C₁-C₆, alcoxy en C₁-C₆, hydroxy, trifluorométhyle, phényle ou benzyloxy; stéréoisomère, isomère géométrique ou isomère optique de celui-ci, ou sel d'addition avec un acide pharmaceutiquement acceptable de celui-ci.

2. Composé selon la revendication 1, dans lequel n est 2.

3. Composé selon la revendication 2, dans lequel R est un atome d'hydrogène et R₁ est un atome d'hydrogène ou un radical phényl-alkyle(C₁-C₆), le fragment phényle étant non substitué ou substitué par 1, 2 ou 3 subtituants, chacun étant indépendamment un atome d'halogène ou un groupe alkyle en C₁-C₆, alcoxy en C₁-C₆, hydroxy, trifluorométhyle, phényle ou benzyloxy.

4. Composé selon la renvendication 3, dans lequel X est un atome d'hydrogène ou un groupe alkyle en C₁-C₆ ou trifluorométhyle.

5. Procédé pour la préparation d'un composé tel que défini dans la revendication 1, comprenant
a) la mise en réaction d'un composé de formule III 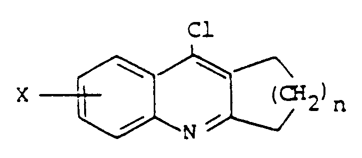 dans laquelle X et n sont tels que définis, avec une hydroxylamine de formule II 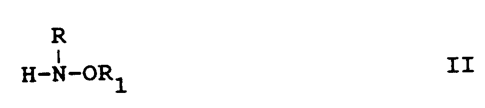 dans laquelle R₁ est un tel que défini et R est tel que défini mais n'est pas un radical alkyl(C₁-C₆)-carbonyle, et
b) éventuellement la mise en réaction d'un composé de formule I, dans lequel R est tel que défini mais n'est pas un radical alkyl(C₁-C₆)-carbonyle, avec un agent d'acylation, pour l'obtention d'un composé de formule I dans lequel R est un radical alkyl(C₁-C₆)-carbonyle, et
éventuellement la préparation, d'une façon usuelle, d'un sel d'addition avec un acide pharmaceutiquement acceptable de celui-ci.

6. Composition pharmaceutique comprenant, en tant que composant actif, un composé tel que défini dans la revendication 1 et un véhicule pharmaceutiquement acceptable.

7. Utilisation d'un composé tel que défini dans la revendication 1, pour la fabrication d'un médicament ayant une activité stimulant la fonction cholinergique.

## Revendications (Revendications pour l'(les) Etat(s) contractant(s) suivant(s): ES, GR)

1. Procédé pour la préparation d'un composé de formule I 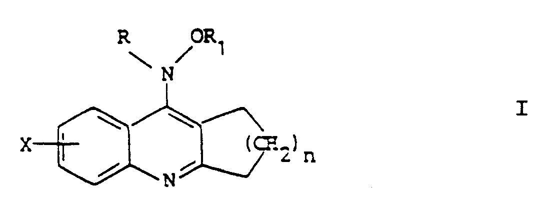 dans laquelle n est 1-4; X est un atome d'hydrogène ou d'halogène ou un radical alkyle en C₁-C₆, cycloalkyle en C₃-C₇, alcoxy en C₁-C₆, hydroxy, nitro, trifluorométhyle, formyle, alkyl(C₁-C₆)-carbonyle, phénylcarbonyle dans lequel le fragment phényle est non substitué ou substitué par 1, 2 ou 3 substituants, chacun étant indépendamment un atome d'halogène ou un groupe alkyle en C₁-C₆, alcoxy en C₁-C₆, hydroxy, trifluorométhyle, phényle ou benzyloxy, un radical -SH, alkyl(C₁-C₆)-thio, NHCOR₂ ou -NR₃R₄, R₂ étant un atome d'hydrogène ou un groupe alkyle en C₁-C₆, et R₃ et R₄ étant indépendamment un atome d'hydrogène ou un groupe alkyle en C₁-C₆ ou cycloalkyle en C₃-C₇; R est un atome d'hydrogène ou un groupe alkyle en C₁-C₆ ou alkyl(C₁-C₆)-carbonyle; et R₁ est un atome d'hydrogène ou un radical alkyle en C₁-C₆, dialkyl(C₁-C₆)-amino-alkyle(C₁-C₆), furyl-alkyle(C₁-C₆), thiényl-alkyle(C₁-C₆), pyridinyl-alkyle(C₁-C₆) ou phényle, phényl-alkyle(C₁-C₆), diphényl-alkyle(C₁-C₆), un radical phényl-alkyle(C₁-C₆) ponté par un atome d'oxygène ou un radical diphényl-alkyle(C₁-C₆) ponté par un atome d'oxygène, le fragment phényle étant non substitué ou substitué par 1, 2 ou 3 substituants, chacun étant indépendamment un atome d'halogène ou un radical alkyle en C₁-C₆, alcoxy en C₁-C₆, hydroxy, trifluorométhyle, phényle ou benzyloxy; d'un stéréoisomère, isomère géométrique ou isomère optique de celui-ci, ou d'un sel d'addition avec un acide pharmaceutiquement acceptable de celui-ci, comprenant
a) la mise en réaction d'un composé de formule III 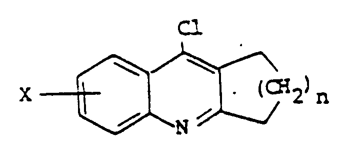 dans laquelle X et n sont tels que définis, avec une hydroxylamine de formule II 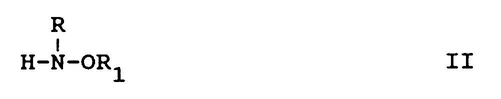 dans laquelle R₁ est un tel que défini et R est tel que défini mais n'est pas un radical alkyl(C₁-C₆)-carbonyle, et
b) éventuellement la mise en réaction d'un composé de formule I, dans lequel R est tel que défini mais n'est pas un radical alkyl(C₁-C₆)-carbonyle, avec un agent d'acylation, pour l'obtention d'un composé de formule I dans lequel R est un radical alkyle(C₁-C₆)-carbonyle, et
éventuellement la préparation, d'une façon usuelle, d'un sel d'addition avec un acide pharmaceutiquement acceptable de celui-ci.

2. Procédé selon la revendication 1, dans lequel n est 2.

3. Procédé selon la revendication 2, dans lequel R est un atome d'hydrogène et R₁ est un atome d'hydrogène ou un radical phényl-alkyle(C₁-C₆).

4. Procédé selon la renvendication 3, dans lequel X est un atome d'hydrogène ou un groupe alkyle en C₁-C₆ ou trifluorométhyle.

5. Utilisation d'un composé selon la revendication 1, pour la fabrication d'un médicament ayant une activité stimulant la fonction cholinergique.
